(19) Europäisches Patentamt
European Patent Office
Office européen des brevets

(11) EP 2 938 321 B1

(12) **EUROPEAN PATENT SPECIFICATION**

(45) Date of publication and mention of the grant of the patent:
**23.08.2017 Bulletin 2017/34**

(21) Application number: **13821484.6**

(22) Date of filing: **23.12.2013**

(51) Int Cl.:
***A61K 8/36*** (2006.01) ***A61K 8/49*** (2006.01)
***A61K 8/60*** (2006.01) ***A61Q 5/06*** (2006.01)
***A61K 8/34*** (2006.01) ***A61K 8/368*** (2006.01)

(86) International application number:
**PCT/EP2013/077937**

(87) International publication number:
**WO 2014/102251 (03.07.2014 Gazette 2014/27)**

(54) **METHOD FOR HAIR COLOURING EMPLOYING A CHROMENE OR CHROMAN DYE AND A SPECIFIC POLYPHENOLIC ACID**

VERFAHREN ZUR HAARFÄRBUNG MIT EINEM CHROMEN- ODER CHROMANFARBSTOFF UND EINER SPEZIFISCHEN POLYPHENOLSÄURE

PROCÉDÉ POUR LA COLORATION DE CHEVEUX UTILISANT UN COLORANT CHROMÈNE OU CHROMANE ET UN ACIDE POLYPHÉNOLIQUE PARTICULIER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.12.2012 FR 1262868**
**25.02.2013 US 201361768579 P**

(43) Date of publication of application:
**04.11.2015 Bulletin 2015/45**

(73) Proprietor: **L'OREAL**
**75008 Paris (FR)**

(72) Inventor: **LALLEMAN, Boris**
**F-75004 Paris (FR)**

(74) Representative: **Casalonga**
**Casalonga & Partners**
**Bayerstraße 71/73**
**80335 München (DE)**

(56) References cited:
**EP-A1- 1 870 081** **EP-A2- 2 196 188**
**WO-A1-2011/086284** **WO-A1-2011/141460**
**CN-A- 102 048 670** **CN-A- 102 397 170**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 938 321 B1

## Description

The present invention relates to a method for colouring keratin fibres that comprises sequential application to the said fibres of a composition (a) comprising one or more dyes chosen from chromene dyes and chroman dyes, in combination with a composition (b) comprising one or more specific polyphenolic acids. The invention also pertains to a composition which is appropriate for the implementation of such a method, and to a kit containing such compositions.

It is familiar art to dye keratin fibres, and more particularly human hair, with dyeing compositions containing oxidation dye precursors, referred to generally as oxidation bases, such as ortho- or para-phenylenediamines, ortho- or para-aminophenols and heterocyclic compounds. These oxidation bases are generally combined with couplers. These bases and couplers are colourless or weakly coloured compounds, which, when combined with oxidizing products, are able to give rise to coloured compounds by a process of oxidative condensation. This type of colouring by oxidation results in permanent colourations, but causes degradation of the keratin fibres as a result of the use of oxidizing agents.

It is also known art to dye keratin fibres, and more particularly the hair, with dyeing compositions comprising direct dyes. The conventional dyes which are used are, in particular, nitrobenzene, anthraquinone, nitropyridine, azo, xanthene, acridine, azine and triarylmethane dyes or natural dyes. These dyes are coloured or colouring molecules which have a certain affinity for keratin fibres. Document CN102048670 discloses a natural and non-toxic method of dyeing hair by applying a composition obtained by mixing haematoxylin and gallic acid. The compositions comprising one or more direct dyes are applied to the keratin fibres for a time required for the desired colouration to be obtained, and are then rinsed. The resulting colourations are particularly chromatic colourations which are, however, temporary or semi-permanent in nature, since the type of interactions that bond the direct dyes to the keratin fibre, and their desorption from the surface and/or core of the fibre, are responsible for their low dyeing power and for their poor resistance to washing or to perspiration.

There is, moreover, increasing demand for hair colouring methods that use natural products.

A genuine need therefore exists to develop hair colouring methods that lead to powerful, bright colourations of low selectivity, which are resistant to external agents (such as, among others, light, adverse weather, shampoos, perspiration), respecting the nature of the hair, on the basis of compositions containing natural dyes. Colourations with greater shampoo resistance are required in particular.

Also increasingly demanded are colouring methods which yield such results in a satisfactory way without the specific need to employ chemical oxidizing agents.

These objectives are achieved by the present invention, which provides a method for colouring keratin fibres, such as human keratin fibres, and more particularly the hair, characterized in that it is carried out in at least two steps, by sequential application to the keratin fibres of:

- a dyeing composition (a) comprising one or more dyes chosen from chromene dyes and chroman dyes, and
- a composition (b) comprising one or more polyphenolic acids chosen from tannic acid, gallic acid, ellagic acid and the salts of these acids.

The present invention additionally provides a dyeing composition and a device comprising a plurality of compartments, or dyeing kit, which is appropriate for the implementation of the method according to the invention.

Other features, aspects and advantages of the present invention will emerge from a reading of the detailed description that is to follow.

The dyes used in the method according to the invention are dyes chosen from chromene dyes and chroman dyes.

According to the invention, the terms "chromene dye" and "chroman dye" mean dyes which comprise in their structure at least one bicyclic system of formula (A) below:

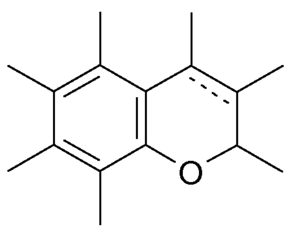

**A**

where the endocyclic bond represents a carbon-carbon single bond or else a carbon-carbon double bond, as

illustrated by formula A1, denoting the class of the chromenes, and by formula A2, denoting the class of the chromans, below:

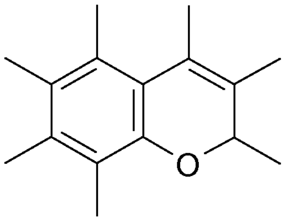

A1

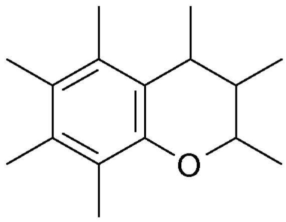

A2

The dyes of formula (A) are chosen more particularly from the compounds of formulae below:

(i) the compounds of formula (I), comprising in their structure the bicyclic system of formula A2,

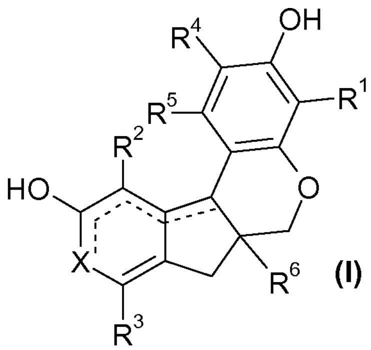

(I)

in which:

- represents a carbon-carbon single bond or a carbon-carbon double bond, the sequence of these bonds denoting two carbon-carbon single bonds and two carbon-carbon double bonds, the said bonds being conjugated,
- X represents a group:

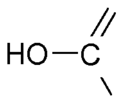

or

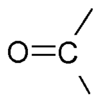

- $R^1$, $R^2$, $R^3$, $R^4$, $R^5$ and $R^6$, which are identical or different, represent, independently of one another, a hydrogen atom, a hydroxyl group, an optionally substituted alkyl or optionally substituted alkoxy group, or an optionally substituted acyloxy group,

and also the tautomeric and/or mesomeric forms thereof, the stereoisomers thereof, the addition salts thereof with a cosmetically acceptable acid or base, and the hydrates thereof, and
(ii) the compounds of formula (II), comprising in their structure the bicyclic system of formula A1:

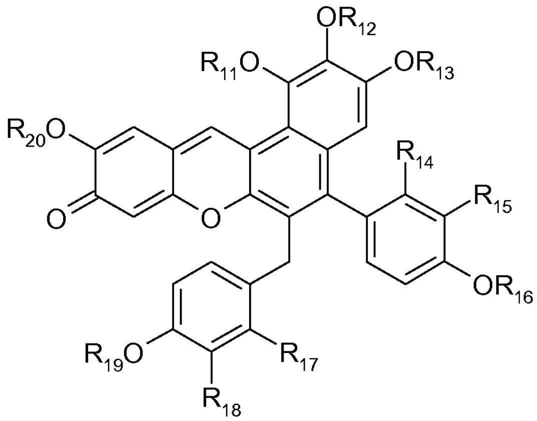

(II)

in which:

- $R_{11}$, $R_{12}$, $R_{13}$, $R_{16}$, $R_{19}$ and $R_{20}$, which are identical or different, represent, independently of one another, a hydrogen atom or a $C_1$-$C_4$ alkyl radical,
- $R_{14}$, $R_{15}$, $R_{17}$ and $R_{18}$, which are identical or different, represent, independently of one another, a hydrogen atom, a hydroxyl radical or a $C_1$-$C_4$ alkoxy radical,

and also the tautomeric and/or mesomeric forms thereof, the stereoisomers thereof, the addition salts thereof with a cosmetically acceptable acid or base, and the hydrates thereof.

With regard to the compounds of formula (I) as defined above, they may be in two tautomeric forms, labelled (Ia) and (Ib):

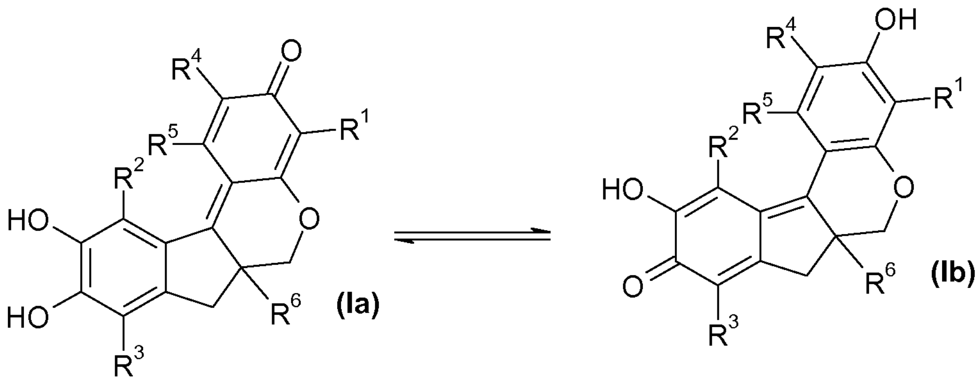

(Ia)

(Ib)

The alkyl radicals referred to in the above definitions of the substituents are generally $C_1$-$C_{20}$, particularly $C_1$-$C_{10}$ and preferably $C_1$-$C_6$, linear or branched, saturated hydrocarbon radicals, such as methyl, ethyl, propyl, butyl, pentyl and hexyl.

The alkoxy radicals are alkyl-oxy radicals with the alkyl radicals as defined above, and the alkoxy radicals are preferably $C_1$-$C_{10}$ radicals, such as methoxy, ethoxy, propoxy and butoxy.

The alkyl or alkoxy radicals, when they are substituted, may be substituted with at least one substituent borne by at least one carbon atom, chosen from:

- a halogen atom;
- a hydroxyl group;
- a $C_1$-$C_2$ alkoxy radical;
- a $C_1$-$C_{10}$ alkoxycarbonyl radical;
- a (poly)-hydroxy-$C_2$-$C_4$ alkoxy radical;
- an amino radical;
- a 5- or 6-membered heterocycloalkyl radical;
- an optionally cationic, 5- or 6-membered heteroaryl radical, preferably imidazolium, which is optionally substituted

by a ($C_1$-$C_4$) alkyl radical, preferably methyl;

- an amino radical substituted by one or two identical or different $C_1$-$C_6$ alkyl radicals which optionally bear at least:

  * one hydroxyl group,
  * an amino group optionally substituted by one or two optionally substituted $C_1$-$C_3$ alkyl radicals, it being possible for the said alkyl radicals, with the nitrogen atom to which they are attached, to form a heterocycle comprising from 5 to 7 chain members, which is saturated or unsaturated and is optionally substituted, optionally comprising at least one other heteroatom the same as or different from nitrogen,
  * a quaternary ammonium group $-N^+R'R''R'''$, $M^-$ for which R', R" and R"', which are identical or different, represent a hydrogen atom or a $C_1$-$C_4$ alkyl group; and $M^-$ represents the counterion of the corresponding organic or inorganic acid or the halide,
  * or a 5- or 6-membered heteroaryl radical which is optionally cationic, preferably imidazolium, and is optionally substituted by a ($C_1$-$C_4$) alkyl radical, preferably methyl;

- an acylamino radical (-NR-COR') in which the radical R is a hydrogen atom or a $C_1$-$C_4$ alkyl radical which optionally bears at least one hydroxyl group, and the radical R' is a $C_1$-$C_2$ alkyl radical;
- a carbamoyl radical ($(R)_2N$-CO-) in which the radicals R, which are identical or non-identical, represent a hydrogen atom or a $C_1$-$C_4$ alkyl radical optionally bearing at least one hydroxyl group;
- an alkylsulfonylamino radical ($R'SO_2$-NR-) in which the radical R represents a hydrogen atom or a $C_1$-$C_4$ alkyl radical optionally bearing at least one hydroxyl group, and the radical R' represents a $C_1$-$C_4$ alkyl radical or a phenyl radical;
- an aminosulfonyl radical ($(R)_2N$-$SO_2$-) in which the radicals R, which are identical or non-identical, represent a hydrogen atom or a $C_1$-$C_4$ alkyl radical which optionally bears at least one hydroxyl group;
- a carboxyl radical in acid or salified form (preferably salified with an alkali metal or a substituted or unsubstituted ammonium);
- a cyano group;
- a nitro group;
- a carboxyl or glycosylcarbonyl group;
- a phenylcarbonyloxy group which is optionally substituted by one or more hydroxyl groups;
- a glycosyloxy group; and
- a phenyl group which is optionally substituted by one or more hydroxyl groups.

The term "glycosyl radical" means a radical derived from a monosaccharide or polysaccharide.

Preferably, the alkyl or alkoxy radicals of formula (I) are unsubstituted.

According to one particular embodiment of the invention, the compounds of formula (I) comprise a radical $R^6$ which represents a hydroxyl group.

Another particular embodiment of the invention concerns the compounds of formula (I) for which the radical $R^1$ represents a hydrogen atom or a hydroxyl group.

More particularly, the method for colouring keratin fibres employs, in the composition (a), one or more dyes of formula (I) chosen from haematoxylin, haematein, brazilin and brazileine.

| | |
|---|---|
| 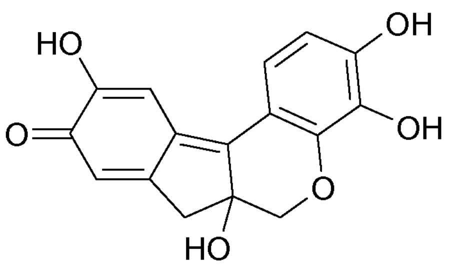 Haematein (oxidized form) | 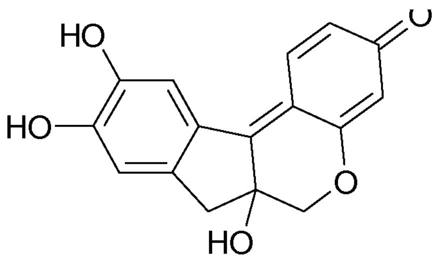 Brazileine (oxidized form) |

(continued)

| | |
|---|---|
| 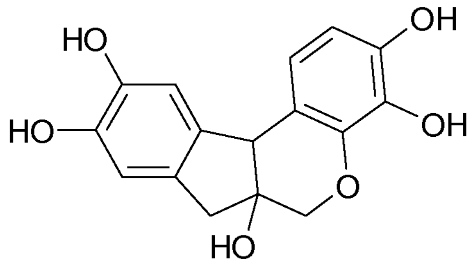 | 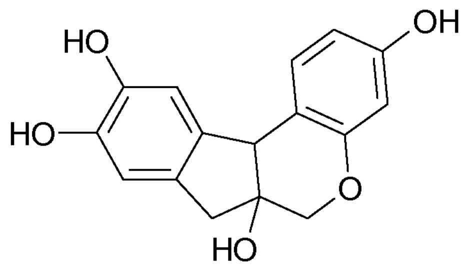 |
| Haematoxylin (Natural Black 1 - CAS 517-28-2) | Brazilin (Natural Red 24 - CAS 474-07-7) |

Brazileine is a conjugated form of a chroman compound of formula A2. The tautomeric structures (Ia) and (Ib) illustrated above are found in the scheme below.

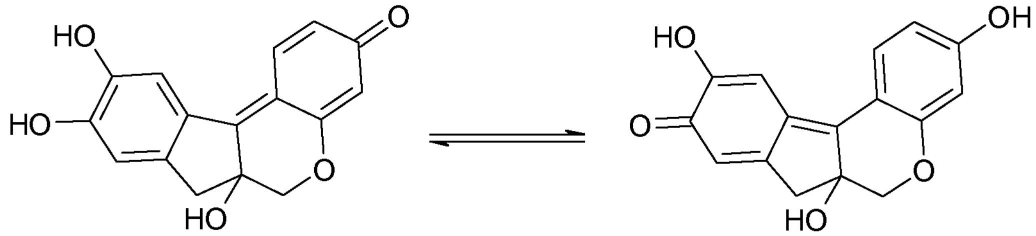

Brazileine

The haematoxylin/haematein and brazilin/brazileine compounds include, by way of example, haematoxylin (Natural Black 1 according to the INCI naming) and brazilin (Natural Red 24 according to the INCI naming), which are compounds from the class of the indochromans, and are available commercially. These compounds may exist in an oxidized form and may be obtained synthetically or by extraction from plants or vegetables known to be rich in these compounds.

The compounds of formula (I) may more particularly be used in the form of extracts. Use may be made of the following plant extracts (genus and species): *Haematoxylon campechianum, Haematoxylon brasiletto, Caesalpinia echinata, Caesalpinia sappan, Caesalpinia spinosa,* and *Caesalpinia brasiliensis.*

The extracts are obtained by extracting the various plant parts, for instance the root, the wood, the bark or the leaves.

According to one particular embodiment of the invention, the natural compounds of formula (I) are derived from logwood, pernambuco wood, sappan wood and brazilwood.

With regard to the compounds of formula (II), the compounds used in the present invention are preferably those for which $R_{11}$ and $R_{13}$ represent an alkyl radical, preferably methyl.

Preferably, $R_{12}$, $R_{16}$, $R_{19}$ and $R_{20}$ denote, independently of one another, a hydrogen atom or an alkyl radical, preferably methyl.

Preferably, $R_{14}$ and $R_{17}$ denote, independently of one another, a hydrogen atom or an alkoxy radical, preferably methoxy.

Preferably, $R_{18}$ and $R_{15}$ denote, independently of one another, a hydrogen atom, a hydroxyl radical or an alkoxy radical, preferably methoxy.

A first particularly preferred class of compounds suitable for the present invention is that of the compounds conforming to formula (II) above for which $R_{12}$, $R_{15}$, $R_{16}$, $R_{17}$, $R_{19}$ and $R_{20}$ each represent a hydrogen atom. $R_{11}$ and $R_{13}$ each represent a methyl radical, and $R_{14}$ represents a methoxy radical.

The preferred compounds from this first class include those for which $R_{18}$ represents a methoxy radical (santalin B) or a hydroxyl radical (santalin A).

A second particularly preferred class of compounds suitable for the present invention is that of the compounds conforming to formula (II) above for which:

- $R_{11}$ and $R_{13}$ each represent a methyl radical,
- $R_{17}$ represents the methoxy radical.

One preferred compound from this second class is that for which, additionally, $R_{19}$ represents a methyl radical, $R_{20}$, $R_{12}$, $R_{14}$, $R_{18}$ and $R_{16}$ each represent a hydrogen atom, and $R_{15}$ represents the hydroxyl radical (santarubin A).

A second preferred compound from this second class is that for which $R_{18}$, $R_{20}$, $R_{12}$, $R_{14}$ and $R_{16}$ represent a hydrogen atom, $R_{15}$ represents a methoxy radical, and $R_{19}$ represents a methyl radical (santarubin B).

A third preferred compound from this second class is that for which $R_{20}$, $R_{12}$, $R_{14}$, $R_{15}$, $R_{16}$ and $R_{19}$ represent hydrogen and $R_{18}$ represents the hydroxyl radical (santarubin C).

Another preferred compound from this second class is that for which $R_{15}$ represents a methoxy radical, and $R_{18}$ and $R_{14}$ represent a hydrogen atom, and $R_{20}$, $R_{12}$, $R_{16}$ and $R_{19}$ represent a methyl radical (tetra-O-methylcantarubin).

The compounds of formula (II) may be used more particularly in the form of extracts. Use may be made of the plant extracts of red woods, generally encompassing the Asian and West African red wood species of the *Pterocarpus* genus and of the *Baphia* genus. These woods are, for example, *Pterocarpus santalinus, Pterocarpus osun, Pterocarpus soyauxii, Pterocarpus erinaceus, Pterocarpus indicus* or else *Baphia nitida.* These woods may also be called padauk, sandalwood, narrawood, camwood or else barwood.

Accordingly, extracts that can be used in the present invention, containing compounds of formula (II), may be obtained, for example, from red sandalwood (*Pterocarpus santalinus*), by aqueous basic extraction, such as the product sold under the trade name Santal Concentré SL 709C by the company Copiaa, or else by means of a solvent extraction of sandalwood powder, such as the product sold under the trade name Santal Poudre SL PP by the same company, Copiaa. Also included is the aqueous-alcoholic extract of red sandalwood in powder form, from the company Alban Muller.

Extracts also suitable for the present invention may be obtained from woods such as camwood (*Baphia nitida*) or else barwood (*Pterocarpus soyauxii, Pterocarpus erinaceus*); the latter is thus fractionated and then ground; a conventional alcoholic extraction or an extraction by percolation is then carried out on this ground material, to give a pulverulent extract which is particularly suitable for the implementation of the present invention.

The salts of the compounds of formula (I) and (II) of the invention may be salts of cosmetically acceptable acids or bases.

The acids can be inorganic or organic. Preferably, the acid is hydrochloric acid, which results in chlorides.

The bases can be inorganic or organic. In particular, the bases are alkali metal hydroxides such as sodium hydroxide, which leads to sodium salts.

The compound or compounds of formula (I) and/or (II) that are employed in the method according to the invention are preferably derived from plant extracts. Use may also be made of mixtures of plant extracts.

The natural extracts according to the invention can be provided in the form of powders or liquids. The extracts of the invention are preferably in powder form.

The chromene or chroman dyes used are preferably chosen from haematein, haematoxylin, brazileine, brazilin, santalin A and mixtures thereof. More preferably still, preference is given to using the dyes of formula (I), and especially haematein, haematoxylin and mixtures thereo f.

The dye or dyes chosen from chromene dyes and chroman dyes are present preferably in an amount of from 0.001% to 20% by weight, relative to the total weight of the composition comprising it or them, preferably from 0.01% to 10% by weight, and more preferably still from 0.1% to 5% by weight.

The method according to the invention also employs a composition (b) comprising one or more polyphenolic acids chosen from tannic acid, gallic acid, ellagic acid and the salts of these acids.

According to one particularly preferred embodiment, the polyphenolic acid is chosen from tannic acid and its salts.

The said polyphenolic acid or acids are preferably present in an amount of from 0.1% to 40% by weight, relative to the total weight of the composition comprising it or them, preferably from 0.5% to 30% by weight, more preferably from 1% to 30% by weight, and more preferably still from 5% to 25% by weight.

The compositions employed in the method according to the invention may independently of one another be in diverse formulational forms, such as a powder, a lotion, a foam or mousse, a cream, a gel, or in any other form appropriate for carrying out dyeing of keratin fibres. They may also be packaged in a pump dispenser without propellant or under pressure in an aerosol dispenser in the presence of a propellant and form a foam or mousse.

These compositions advantageously comprise water, a mixture of water and one or more organic solvents or else a mixture of organic solvents, when the compositions are in liquid form.

According to one particular embodiment of the invention, the compositions according to the invention comprise water.

Organic solvents include, for example, lower $C_1$-$C_4$ alkanols, such as ethanol and isopropanol; polyols and polyol ethers such as 2-butoxyethanol, propylene glycol, propylene glycol monomethyl ether, diethylene glycol monoethyl ether and monomethyl ether, and hexylene glycol.

According to a preferred embodiment, at least one of the compositions of the invention also comprises one or more liquid organic compounds having a Hansen solubility parameter $\delta H$ of less than or equal to 16 $Mpa^{1/2}$, preferably strictly less than 16 $Mpa^{1/2}$.

These compounds are liquid at a temperature of 25°C and at atmospheric pressure (760 mmHg).

The organic compound or compounds having a Hansen solubility parameter δH as defined above are described in, for example, the reference work "Hansen solubility parameters A user's handbook, Charles M. Hansen", CRC Press, 2000, pages 167 to 185, or else in "Handbook of Solubility Parameters and other cohesion parameters", CRC Press, pages 95 to 121 and pages 177 to 185.

This solubility parameter δH is associated with the formation of hydrogen bonds.

More particularly, "Handbook of Solubility Parameters and other cohesion parameters", CRC Press, pages 95 to 121 and pages 177 to 185, gives the equation $\delta H = (\Sigma\text{-}{}^{z}U_h/V)^{1/2}$
in which:

> ${}^{z}U_h$ (in $J.mol^{-1}$) describes the contributions of the functional group in question in the solubility parameters linked to the hydrogen bonds (values in table 14, page 183); this parameter ${}^{z}U_h$ is also described in the following work: "The relation between surface tension and solubility parameter in liquids", Bagda, E, Farbe Lack, 84, 212, 1978;
>
> and V is the volume of the molecule.

The value of the solubility parameter δH is usually given for a temperature of 25°C.

The said liquid organic compound or compounds may be chosen from alkanols, aliphatic esters, ethers, aromatic alcohols, alkylaryl alcohols, aromatic acids, aliphatic acids, alkylene carbonates such as propylene carbonate, lactones such as γ-butyrolactone, and mixtures thereo f.

The said liquid organic compound or compounds are preferably chosen from benzyl alcohol, phenylpropanol, phenylethanol, phenoxyethanol, linear alcohols containing from 5 to 12 carbon atoms (and, among the latter, more preferably pentanol, octanol, decanol, and mixtures thereof), and mixtures thereof.

The said liquid organic compound or compounds may be present in proportions preferably ranging from 1% to 40% by weight, relative to the total weight of each composition comprising it or them, and more preferably from 2% to 20% by weight.

According to another embodiment of the invention, at least one of the compositions used in the method of the invention is anhydrous and may be in a pulverulent or pasty form.

When the composition is in pulverulent form, it may contain pulverulent ingredients.

When the composition is in the form of a paste, it may optionally contain one or more inert organic liquids, preferably chosen from liquid petrolatum, polydecenes and fatty esters which are liquid at ambient temperature (25°C) and at atmospheric pressure (760 mmHg, or 1.013 bar).

According to one embodiment which is also preferred, at least one of the compositions according to the invention also comprises one or more metal salts and/or metal oxides.

The metal oxide or oxides and/or salt or salts are preferably chosen from the salts and/or oxides of zinc, manganese, iron, copper or aluminium.

The salts are particularly preferred. The salts include the halides such as chlorides, fluorides and iodides; sulfates and phosphates; nitrates; perchlorates and carboxylic salts and polymeric salts, and also mixtures thereof.

Carboxylic salts which may be used in the invention also include hydroxycarboxylic salts such as gluconate, or amino carboxylic salts such as glycinate.

Examples of polymeric salts include manganese pyrrolidonecarboxylate.

Non-limiting examples of manganese salts include manganese chloride, manganese fluoride, manganese acetate tetrahydrate, manganese lactate trihydrate, manganese phosphate, manganese iodide, manganese nitrate trihydrate, manganese bromide, manganese perchlorate tetrahydrate, manganese sulfate monohydrate, manganese gluconate, and manganese glycinate. The manganese salts used advantageously are manganese glycinate and manganese acetate.

The salts of zinc, iron, copper or aluminium include sulfates, gluconates, chlorides, lactates, acetates, glycinates, aspartates and citrates.

The metal oxide or oxides and/or salt or salts may be introduced in solid form into the compositions or else may come from a natural water, mineral or thermal, which is rich in these ions, or else from seawater (Dead Sea especially). They may also originate from mineral compounds, for instance earths, ochres such as clays (green clay, for example) or even from a plant extract containing them (cf. for example, patent document FR 2 814 943).

The metal oxides and/or salts of the invention are preferably of oxidation state 2.

The particularly preferred metal salt or salts are chosen from zinc glycinate, manganese glycinate, manganese chloride, iron sulfate and mixtures thereof.

According to a preferred embodiment of the invention, the metal oxide or oxides and/or salt or salts employed represent from 0.001% to 10% by weight of the total weight of the composition or compositions containing this or these metal oxides and/or salts, and more preferably still from 0.05% to 5% by weight.

The method according to the invention exhibits, furthermore, the advantage of providing excellent results,

including when it is implemented in the absence of a chemical oxidizing agent.

Accordingly, according to one embodiment, the method according to the invention does not employ a chemical oxidant, i.e. an oxidizing agent other than atmospheric oxygen.

A chemical oxidizing agent denotes more particularly hydrogen peroxide, urea peroxide, alkali metal bromides and persalts such as perborates and persulfates.

In this embodiment, none of the compositions of the invention contains a chemical oxidizing agent.

The compositions employed in the colouring method in accordance with the invention may also comprise various adjuvants conventionally used in compositions for dyeing hair, such as anionic, cationic, nonionic, amphoteric or zwitterionic surfactants or mixtures thereof, anionic, cationic, nonionic, amphoteric or zwitterionic polymers or mixtures thereof, organic or inorganic thickeners, and more particularly anionic, cationic, nonionic and amphoteric polymeric associative thickeners, antioxidants, sequestrants, fragrances, buffers, dispersants, conditioning agents such as modified or non-modified, volatile or non-volatile silicones, film formers, ceramides, preservatives and opacifiers.

The said adjuvants are preferably chosen from surfactants such as anionic and nonionic surfactants or mixtures thereof and from organic or inorganic thickeners.

The adjuvants above are generally present in an amount, for each of them, of between 0.01% and 40% by weight, relative to the weight of the composition or compositions comprising them, preferably between 0.1% and 20% by weight, relative to the weight of the composition or compositions comprising them.

Needless to say, those skilled in the art will take care to select this or these optional additional compounds such that the advantageous properties intrinsically associated with the composition that are of use in the dyeing process in accordance with the invention are not, or are not substantially, adversely affected by the envisaged addition(s).

The method according to the invention may employ one or more additional dyes, which may be present within either of the compositions according to the invention.

These additional dyes may more particularly be direct dyes, which are chosen, for example, from those conventionally used in direct dyeing, and which include all of the aromatic and/or non-aromatic dyes in current use, such as neutral, acidic or cationic nitrobenzene direct dyes, neutral, acidic or cationic azo direct dyes, neutral, acidic or cationic quinone, and more particularly anthraquinone, direct dyes, azine, triarylmethane and indoamine direct dyes, methines, styryls, porphyrins, metalloporphyrins, phthalocyanines, methine cyanines, fluorescent dyes, and natural dyes other than the chromans and chromenes.

The natural direct dyes include lawsone, juglone, indigo, isatin, curcumin, spinulosin, apigenidin and orceins. Use may also be made of extracts or decoctions comprising these natural dyes and in particular henna-based poultices or extracts.

The additional dye or dyes represent preferably from 0.001% to 10% by weight, approximately, of the total weight of the composition or compositions comprising it or them.

When the chromene and/or chroman dye or dyes are present in an aqueous composition, the pH of this aqueous composition varies advantageously from 2 to 12. This pH is preferably neutral, meaning that it varies from 6 to 8, and more preferably from 6.5 to 7.5.

This pH may be adjusted to the desired value by means of acidifying or alkalifying agents which are commonly used in the dyeing of keratin fibres, or else by means of conventional buffer systems.

The acidifying agents include, for example, inorganic or organic acids such as hydrochloric acid, orthophosphoric acid, sulfuric acid, carboxylic acids such as acetic acid, tartaric acid, citric acid and lactic acid, and sulfonic acids.

The alkalifying agent or agents may more particularly be chosen from aqueous ammonia, alkali metal carbonates, alkanolamines such as mono-, di- and triethanolamines and also derivatives thereof, sodium hydroxide or potassium hydroxide, and the compounds of the formula below:

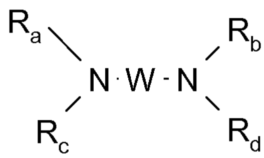

in which W is a propylene residue optionally substituted by a hydroxyl group or a $C_1$-$C_4$ alkyl radical; $R_a$, $R_b$, $R_c$ and $R_d$, which are identical or different, represent a hydrogen atom or a $C_1$-$C_4$ alkyl or $C_1$-$C_4$ hydroxyalkyl radical.

The colouring method is carried out in at least two steps, by sequential application to the keratin fibres of the compositions (a) and (b) as defined above.

These steps may or may not be separated by interim rinsing. In the absence of such interim rinsing, wringing may be carried out, with a towel or with paper, so as to remove the excess composition.

In a first variant, the method according to the invention comprises a first step of applying to said fibres a dyeing composition (a) as defined above, then a second step of applying to said fibres a composition (b) as described above.

In a second variant, the method according to the invention comprises a first step of applying to said fibres a composition (b) as described above, then a second step of applying to said fibres a dyeing composition (a) as described above.

The first variant above is preferred.

For both variants, the leave-on time after application of the composition for the first step is generally from 3 to 120 minutes, preferably from 10 to 60 minutes, and more preferably from 15 to 45 minutes. The leave-on time after application of the composition for the second step is generally from 3 to 120 minutes, preferably from 3 to 60 minutes, and more preferably from 5 to 30 minutes.

The temperature at which the compositions of the invention are applied is generally between room temperature (15 to 25°C) and 80°C and more particularly between 15 and 45°C. Hence, following application of the composition according to the invention, it is possible, advantageously, to subject the hair to thermal treatment by heating at a temperature of between 30 and 60°C. In practice, this operation may be conducted using a styling hood, a hairdryer, an infra-red radiation dispenser, and other conventional heating appliances.

It is also possible, both as heating means and as a means for smoothing the hair, to use heating tongs, at a temperature between 60 and 220°C and preferably between 120 and 200°C.

The invention further pertains to a two-part cosmetic composition comprising:

- a dyeing composition (a) as described above, and comprising one or more dyes chosen from chromene dyes and chroman dyes, and
- a composition (b) as described above, and comprising one or more polyphenolic acids chosen from tannic acid, gallic acid, ellagic acid and the salts of these acids.

The composition according to the invention is particularly appropriate for the implementation of the method of the invention as described above, and it may include any additional compounds, such as, among others, those described above.

The invention further pertains to the use of the compositions according to the invention for colouring keratin fibres such as human keratin fibres, and more particularly the hair.

The composition according to the invention may advantageously be packaged in a kit, in other words a device having a plurality of compartments.

Thus, the present invention additionally provides a kit or device for the dyeing of keratin fibres, comprising at least two compartments:

- a first compartment containing a dyeing composition (a) comprising one or more dyes chosen from chromene dyes and chroman dyes; and
- a second compartment containing a composition (b) comprising one or more polyphenolic acids chosen from tannic acid, gallic acid, ellagic acid and the salts of these acids.

According to one variant of the invention, the kit further comprises an additional composition comprising one or more treatment agents. The compositions in the kit are packaged in separate compartments, which may optionally be accompanied by suitable, identical or different, application means, such as fine or coarse brushes or sponges.

The abovementioned kit may also be equipped with means allowing the delivery to the hair of the desired mixture, such as, for example, the device described in patent FR 2 586 913.

The evaluation of the colouration can be done visually or read on a spectrocolorimeter (such as Minolta CM3600d, illuminant D65, angle 10°, SCI values) for the L*, a*, b* colorimetric measurements. In this L*, a*, b* system, L* represents the intensity of the colour, a* indicates the green/red colour axis and b* indicates the blue/yellow colour axis. The lower the value of L, the darker or more intense the colour. The higher the value of a*, the redder the shade; the higher the value of b*, the yellower the shade. The variation in colouring between the coloured locks of natural white hair (NW) which is untreated (control) and after treatment or colouration is defined by ΔE*, corresponding to the colour uptake on keratin fibres, according to the following equation:

$$\Delta E^* = \sqrt{(L^* - L_o^*)^2 + (a^* - a_o^*)^2 + (b^* - b_o^*)^2}$$

In this equation, L*, a* and b* represent the values measured after dyeing the natural hair comprising 90% of white hairs and $L_0^*$, $a_0^*$ and $b_0^*$ represent the values measured for the untreated natural hair comprising 90% of white hairs.

The greater the value of ΔE, the greater the difference in colour between the control locks and the dyed locks and the greater colour uptake is.

On the other hand, for evaluating the selectivity of the colour between the root and tip of the keratin fibre, measurement can be done on permed or sensitized white hair (PW) and natural white hair, wherein the variation in colouring between the coloured locks PW and the coloured natural white hair as defined by ΔE*, corresponding to the selectivity of the colour, is calculated according to the following equation:

$$\Delta E^* = \sqrt{(L^* - L_o^*)^2 + (a^* - a_o^*)^2 + (b^* - b_o^*)^2}$$

In this equation, L*, a* and b* represent the values measured after dyeing the natural hair comprising 90% of white hairs and $L_0$*, $a_0$* and $b_0$* represent the values measured after dyeing the permed or sensitized hair. The lowest ΔE* is the best homogeneity of the hair colour.

If the light fastness is investigated, ΔE* is also calculated for the $L_0$*, $a_0$*, $b_0$* and L*, a*, b* measured of the locks before and after exposure to the light, respectively.

Chromaticity in the CIE L*, a*, b* colourimetric system is calculated according to the following equation:

$$C^* = \sqrt{a^{*2} + b^{*2}}$$

The greater the value of C*, the greater the chromaticity is.

The examples that follow serve to illustrate the invention without, however, being limiting in nature.

EXAMPLE:

Compositions (a) and (b) below are prepared from the ingredients detailed hereinafter, the proportions of which are indicated in grams.

| | Composition (a) |
|---|---|
| Oxidized logwood extract containing 9% haematein and 5% haematoxylin | 4 g |
| Benzyl alcohol | 4.8 g |
| Ethanol | 14.4 g |
| Bentone | 3.8 g |
| Fragrance | qs |
| Water | qs 100 g |

| | Composition (b) |
|---|---|
| Tannic acid | 20 g |
| Ethanol | 9.7 g |
| Water | qs 100 g |

Pairs of locks of natural and permed Caucasian hair containing 90% white hairs are treated in succession with:

1. the composition (a), which is left on for 45 minutes at 40°C, followed by rinsing and wringing, and then
2. the composition (b), which is left on for 15 minutes at 40°C.

Following the application of these treatments, the locks are rinsed, wrung and dried.

Locks with an intense copper red colouration are obtained which are very shiny.

The hair then undergoes a test for tenacity after nine shampooings.

The hair coloured using the method according to the invention is found to exhibit a high level of shampoo tenacity, the colour retaining a high level of intensity and shine.

## Claims

1. Method for colouring keratin fibres, such as human keratin fibres, and more particularly the hair, **characterized in that** it is carried out in at least two steps, by sequential application to the keratin fibres of:

   - a dyeing composition (a) comprising one or more dyes chosen from chromene dyes and chroman dyes, and
   - a composition (b) comprising one or more polyphenolic acids chosen from tannic acid, gallic acid, ellagic acid and the salts of these acids.

2. Method according to Claim 1, wherein the dye or dyes chosen from chromene dyes and chroman dyes are chosen from the compounds of formulae below:

   (i) the compounds of formula (I):

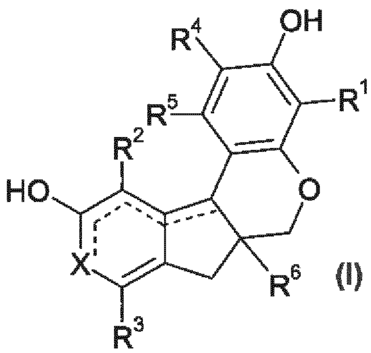

(I)

in which:

- ⎓ represents a carbon-carbon single bond or a carbon-carbon double bond, the sequence of these bonds ⎓ denoting two carbon-carbon single bonds and two carbon-carbon double bonds, the said bonds being conjugated,
- X represents a group:

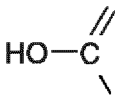 or 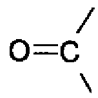

- $R^1$, $R^2$, $R^3$, $R^4$, $R^5$ and $R^6$, which are identical or different, represent, independently of one another, a hydrogen atom, a hydroxyl group, an optionally substituted alkyl or optionally substituted alkoxy group, or an optionally substituted acyloxy group,

and also the tautomeric and/or mesomeric forms thereof, the stereoisomers thereof, the addition salts thereof with a cosmetically acceptable acid or base, and the hydrates thereof, and

(ii) the compounds of formula (II):

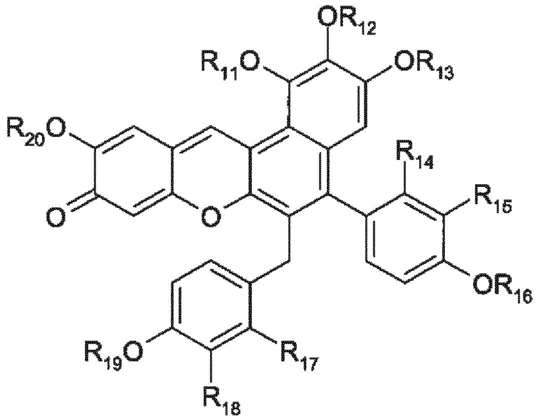

(II)

in which:

- $R_{11}$, $R_{12}$, $R_{13}$, $R_{16}$, $R_{19}$ and $R_{20}$, which are identical or different, represent, independently of one another, a hydrogen atom or a $C_1$-$C_4$ alkyl radical,
- $R_{14}$, $R_{15}$, $R_{17}$ and $R_{18}$, which are identical or different, represent, independently of one another, a hydrogen atom, a hydroxyl radical or a $C_1$-$C_4$ alkoxy radical,

and also the tautomeric and/or mesomeric forms thereof, the stereoisomers thereof, the addition salts thereof with a cosmetically acceptable acid or base, and the hydrates thereof.

**3.** Method according to either of the preceding claims, wherein the dye or dyes chosen from the chromene dyes and the chroman dyes are chosen from haematein, haematoxylin, brazileine, brazilin, santalin A and mixtures thereof, and more preferably from haematein, haematoxylin and mixtures thereof.

**4.** Method according to any one of the preceding claims, wherein the dye or dyes chosen from the chromene dyes and chroman dyes represent from 0.001% to 20% by weight, relative to the total weight of the composition comprising it or them, preferably from 0.01% to 10% by weight, and more preferably still from 0.1% to 5% by weight.

**5.** Method according to any one of the preceding claims, wherein the polyphenolic acid is chosen from tannic acid and salts thereof.

**6.** Method according to any one of the preceding claims, wherein the polyphenolic acid or acids represent from 0.1% to 40% by weight, relative to the total weight of the composition comprising it or them, preferably from 0.5% to 30% by weight, more preferably from 1% to 30% by weight, and more preferably still from 5% to 25% by weight.

**7.** Method according to any one of the preceding claims, wherein at least one of the compositions employed comprises water.

**8.** Method according to any one of the preceding claims, wherein at least one of the compositions employed also comprises one or more liquid organic compounds having a Hansen solubility parameter $\delta H$ of less than or equal to 16 $Mpa^{1/2}$, preferably strictly less than 16 $Mpa^{1/2}$, preferably chosen from benzyl alcohol, phenylpropanol, phenylethanol, phenoxyethanol, linear alcohols containing from 5 to 12 carbon atoms, and mixtures thereof.

**9.** Method according to the preceding claim, wherein the said liquid organic compound or compounds are present in proportions ranging from 1% to 40% by weight, relative to the total weight of each composition comprising it or them, and more preferably from 2% to 20% by weight.

**10.** Method according to any one of the preceding claims, wherein at least one of the compositions employed also comprises one or more metal salts and/or metal oxides, chosen preferably from the salts and/or oxides of zinc, manganese, iron, copper or aluminium, and more preferably from zinc glycinate, manganese glycinate, manganese chloride, iron sulfate and mixtures thereof.

**11.** Method according to the preceding claim, wherein the metal salt or salts and/or metal oxide or oxides used represent

from 0.001% to 10% by weight of the total weight of the composition or compositions comprising this or these metal salt or salts and/or metal oxide or oxides, and more preferably still from 0.05% to 5% by weight.

**12.** Method according to any one of the preceding claims, wherein none of the compositions employed contains a chemical oxidizing agent.

**13.** Method according to any one of the preceding claims, comprising a first step of applying to the keratin fibres the dyeing composition (a), then a second step of applying to the keratin fibres the composition (b).

**14.** Kit for dyeing keratin fibres, comprising at least two compartments:

- a first compartment containing a dyeing composition (a) comprising one or more dyes chosen from chromene dyes and chroman dyes; and
- a second compartment containing a composition (b) comprising one or more polyphertolic acids chosen from tannic acid, gallic acid, ellagic acid and the salts of these acids.

**Patentansprüche**

**1.** Verfahren zum Färben von Keratinfasern, wie menschlichen Keratinfasern, und insbesondere des Haars, **dadurch gekennzeichnet, dass** es in mindestens zwei Schritten durchgeführt wird, indem man auf die Keratinfasern nacheinander:

- eine Färbezusammensetzung (a), die einen oder mehrere aus Chromen-Farbstoffen und Chroman-Farbstoffen ausgewählte Farbstoffe umfasst, und
- eine Zusammensetzung (b), die eine oder mehrere Polyphenolsäuren, die aus Tanninsäure, Gallussäure, Ellagsäure und den Salzen dieser Säuren ausgewählt sind, umfasst,

aufbringt.

**2.** Verfahren nach Anspruch 1, bei dem der bzw. die Farbstoffe, die aus Chromen-Farbstoffen und Chroman-Farbstoffen ausgewählt ist bzw. sind, aus den Verbindungen der nachstehenden Formeln ausgewählt ist bzw. sind:

(i) den Verbindungen der Formel (I):

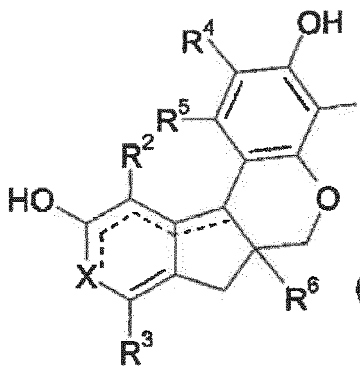

(I)

in der:

- ---- für eine Kohlenstoff-Kohlenstoff-Einfachbindung oder eine Kohlenstoff-Kohlenstoff-Doppelbindung steht, wobei die Sequenz dieser Bindungen ---- zwei Kohlenstoff-Kohlenstoff-Einfachbindungen und zwei Kohlenstoff-Kohlenstoff-Doppelbindungen anzeigt, wobei die Bindungen konjugiert sind,
- X für eine Gruppe:

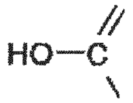 oder 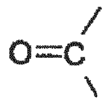

steht,

- $R^1$, $R^2$, $R^3$, $R^4$, $R^5$ und $R^6$, die gleich oder verschieden sind, unabhängig voneinander für ein Wasserstoffatom, eine Hydroxylgruppe, eine gegebenenfalls substituierte Alkylgruppe, eine gegebenenfalls substituierte Alkoxygruppe oder eine gegebenenfalls substituierte Acyloxygruppe stehen,

sowie den tautomeren und/oder mesomeren Formen davon, den Stereoisomeren davon, den Additionssalzen davon mit einer kosmetisch unbedenklichen Säure oder Base und den Hydraten davon und
(ii) den Verbindungen der Formel (II):

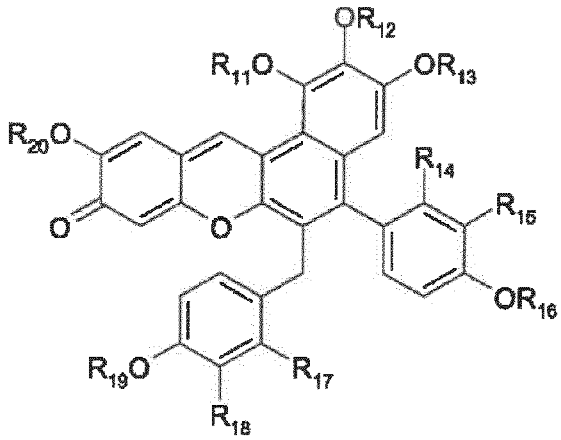

(II)

in der:

- $R_{11}$, $R_{12}$, $R_{13}$, $R_{16}$, $R_{19}$ und $R_{20}$, die gleich oder verschieden sind, unabhängig voneinander für ein Wasserstoffatom oder einen $C_1$-$C_4$-Alkylrest stehen,
- $R_{14}$, $R_{15}$, $R_{17}$ and $R_{18}$, die gleich oder verschieden sind, unabhängig voneinander für ein Wasserstoffatom, einen Hydroxylrest oder einen $C_1$-$C_4$-Alkoxyrest stehen,

sowie den tautomeren und/oder mesomeren Formen davon, den Stereoisomeren davon, den Additionssalzen davon mit einer kosmetisch unbedenklichen Säure oder Base und den Hydraten davon.

**3.** Färbeverfahren nach einem der vorhergehenden Ansprüche, bei dem der bzw. die Farbstoffe, die aus Chromen-Farbstoffen und Chroman-Farbstoffen ausgewählt ist bzw. sind, aus Hämatein, Hämatoxylin, Brasilein, Brasilin, Santalin A und Mischungen davon und weiter bevorzugt aus Hämatein, Hämatoxylin und Mischungen davon ausgewählt ist bzw. sind.

**4.** Färbeverfahren nach einem der vorhergehenden Ansprüche, bei dem der bzw. die Farbstoffe, die aus Chromen-Farbstoffen und Chroman-Farbstoffen ausgewählt ist bzw. sind, 0,001 bis 20 Gew.-%, bezogen auf das Gesamtgewicht der Zusammensetzung, die ihn bzw. sie umfasst, vorzugsweise von 0,01 bis 10 Ges.-% und noch weiter bevorzugt von 0,1 bis 5 Gew.-% ausmacht bzw. ausmachen.

**5.** Verfahren nach einem der vorhergehenden Ansprüche, bei dem die Polyphenolsäure aus Tanninsäure und Salzen davon ausgewählt ist.

**6.** Verfahren nach einem der vorhergehenden Ansprüche, bei dem die Polyphenolsäure bzw. Polyphenolsäuren 0,1 bis 40 Ges.-%, bezogen auf das Gesamtgewicht der Zusammensetzung, die sie umfasst, vorzugsweise 0,5 bis 30 Gew.-%, weiter bevorzugt 1 bis 30 Gew.-% und noch weiter bevorzugt 5 bis 25 Gew.-% ausmacht bzw. ausmachen.

**7.** Verfahren nach einem der vorhergehenden Ansprüche, bei dem mindestens eine der eingesetzten Zusammensetzungen Wasser umfasst.

8. Verfahren nach einem der vorhergehenden Ansprüche, bei dem mindestens eine der eingesetzten Zusammensetzungen außerdem eine oder mehrere flüssige organische Verbindungen, die einen Hansen-Löslichkeitsparameter δH kleiner gleich 16 $Mpa^{1/2}$, vorzugsweise streng weniger als 16 $Mpa^{1/2}$, aufweist bzw. aufweisen und vorzugsweise aus Benzylalkohol, Phenylpropanol, Phenylethanol, Phenoxyethanol, linearen Alkoholen mit 5 bis 12 Kohlenstoffatomen und Mischungen davon ausgewählt ist bzw. sind, umfasst.

9. Verfahren nach dem vorhergehenden Anspruch, bei dem die flüssige organische Verbindung bzw. die flüssigen organischen Verbindungen in Anteilen im Bereich von 1 bis 40 Gew.-%, bezogen auf das Gesamtgewicht jeder Zusammensetzung, die sie enthält, und noch weiter bevorzugt 2 bis 20 Gew.-%, vorliegt bzw. vorliegen.

10. Verfahren nach einem der vorhergehenden Ansprüche, bei dem mindestens eine der eingesetzten Zusammensetzungen außerdem ein oder mehrere Metallsalze und/oder Metalloxide, die vorzugsweise aus den Salzen und/oder Oxiden von Zink, Mangan, Eisen, Kupfer oder Aluminium und weiter bevorzugt aus Zinkglycinat, Manganglycinat, Manganchlorid, Eisensulfat und Mischungen davon ausgewählt sind, umfasst.

11. Verfahren nach dem vorhergehenden Anspruch, bei dem das verwendete Metallsalz bzw. die verwendeten Metallsalze und/oder das verwendete Metalloxid bzw. die verwendeten Metalloxide 0,001 bis 10 Gew.-% des Gesamtgewichts der Zusammensetzung bzw. Zusammensetzungen, die dieses Metallsalz bzw. diese Metallsalze und/oder dieses Metalloxid bzw. diese Metalloxide umfasst bzw. umfassen, und noch weiter bevorzugt 0,05 bis 5 Gew.-% ausmachen.

12. Verfahren nach einem der vorhergehenden Ansprüche, bei dem keine der eingesetzten Zusammensetzungen ein chemisches Oxidationsmittel enthält.

13. Verfahren nach einem der vorhergehenden Ansprüche, das einen ersten Schritt, bei dem man die Färbezusammensetzung (a) auf die Keratinfasern aufbringt, und dann einen zweiten Schritt, bei dem man die Zusammensetzung (b) auf die Keratinfasern aufbringt, umfasst.

14. Kit zum Färben von Keratinfasern mit mindestens zwei Kompartimenten:

- einem ersten Kompartiment, das eine Färbezusammensetzung (a) enthält, die einen oder mehrere aus Chromen-Farbstoffen und Chroman-Farbstoffen ausgewählte Farbstoffe umfasst; und
- einem zweiten Kompartiment, das eine Zusammensetzung (b) enthält, die eine oder mehrere Polyphenolsäuren, die aus Tanninsäure, Gallussäure, Ellagsäure und den Salzen dieser Säuren ausgewählt sind, umfasst.

## Revendications

1. Procédé de coloration de fibres kératiniques, telles que des fibres kératiniques humaines et en particulier des cheveux, **caractérisé en ce qu'**on le réalise en au moins deux étapes, en appliquant successivement sur les fibres kératiniques :

- une composition tinctoriale (a) comprenant un ou plusieurs colorant(s) choisi(s) parmi les colorants chroméniques et les colorants chromaniques,
- et une composition (b) comprenant un ou plusieurs acide(s) polyphénolique(s) choisi(s) parmi l'acide tannique, l'acide gallique, l'acide ellagique et les sels de ces acides.

2. Procédé selon la revendication 1, dans lequel le ou les colorant(s) choisi(s) parmi les colorants chroméniques et les colorants chromaniques est ou sont choisi(s) parmi les composés de formules suivantes :

i) les composés de formule (I) :

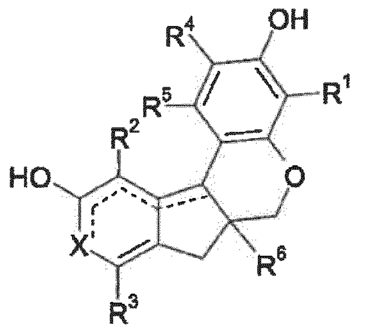

(I)

dans laquelle

- ---- représente une simple liaison carbone-carbone ou une double liaison carbone-carbone, l'enchaînement de ces liaisons ---- désignant deux simples liaisons carbone-carbone et deux doubles liaisons carbone-carbone, lesdites liaisons étant conjuguées,
- X représente un groupement de formule :

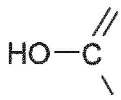 ou 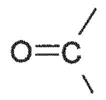 ,

- $R^1$, $R^2$, $R^3$, $R^4$, $R^5$ et $R^6$, identiques ou différents, représentent indépendamment les uns des autres, un atome d'hydrogène, un groupe hydroxyle, un groupe alkyle éventuellement substitué, un groupe alcoxy éventuellement substitué, ou un groupe acyloxy éventuellement substitué,

ainsi que leurs formes tautomères et/ou mésomères, leurs stéréoisomères, leurs sels d'addition d'acide ou de base cosmétiquement acceptable, et leurs hydrates ;
ii) et les composés de formule (II) :

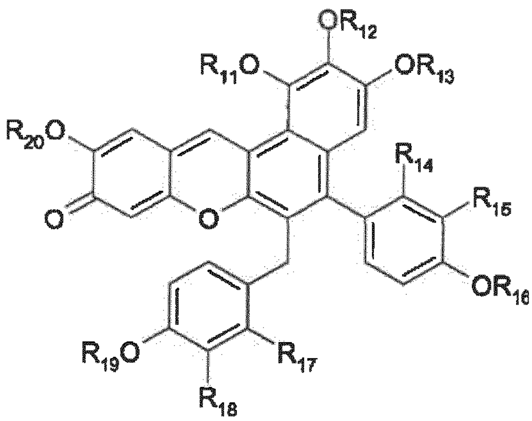

(II)

dans laquelle

- $R_{11}$, $R_{12}$, $R_{13}$, $R_{16}$, $R_{19}$ et $R_{20}$, identiques ou différents, représentent indépendamment les uns des autres, un atome d'hydrogène ou un radical alkyle en $C_1$-$C_4$,
- $R_{14}$, $R_{15}$, $R_{17}$ et $R_{19}$, identiques ou différents, représentent indépendamment les uns des autres, un atome d'hydrogène, un radical hydroxyle ou un radical alcoxy en $C_1$-$C_4$,

ainsi que leurs formes tautomères et/ou mésomères, leurs stéréoisomères, leurs sels d'addition d'acide ou de base cosmétiquement acceptable, et leurs hydrates.

**3.** Procédé selon l'une ou l'autre des revendications précédentes, dans lequel le ou les colorant(s) choisi(s) parmi les

colorants chroméniques et les colorants chromaniques est ou sont choisi(s) parmi l'hématéine, l'hématoxyline, la braziléine, la braziline, la santaline A et leurs mélanges, et de préférence, parmi l'hématéine, l'hématoxyline et leurs mélanges.

**4.** Procédé selon l'une quelconque des revendications précédentes, dans lequel le ou les colorant(s) choisi(s) parmi les colorants chroméniques et les colorants chromaniques représente(nt) de 0,001 % à 20 % en poids, par rapport au poids total, de la composition le ou les contenant, de préférence de 0,01 à 10 % en poids, et mieux encore de 0,1 à 5 % en poids.

**5.** Procédé selon l'une quelconque des revendications précédentes, dans lequel l'acide polyphénolique est choisi parmi l'acide tannique et ses sels.

**6.** Procédé selon l'une quelconque des revendications précédentes, dans lequel le ou les acide(s) polyphénolique(s) représente(nt) de 0,1 à 40 % en poids, par rapport au poids total, de la composition le ou les contenant, de préférence de 0,5 à 30 % en poids, plus préférentiellement de 1 à 30 % en poids, et mieux encore de 5 à 25 % en poids.

**7.** Procédé selon l'une quelconque des revendications précédentes, dans lequel au moins l'une des compositions mises en oeuvre contient de l'eau.

**8.** Procédé selon l'une quelconque des revendications précédentes, dans lequel au moins l'une des compositions mises en oeuvre contient également un ou plusieurs composé(s) organique(s) liquide(s) présen-tant un paramètre de solubilité de Hansen δH d'une valeur inférieure ou égale à 16 $Mpa^{1/2}$, de préférence strictement inférieure à 16 $Mpa^{1/2}$, et de préférence choisi(s) parmi l'alcool benzylique, le phényl-propanol, le phényl-éthanol, le phénoxy-éthanol, les alcools linéaires contenant de 5 à 12 atomes de carbone, et leurs mélanges.

**9.** Procédé selon la revendication précédente, dans lequel ledit ou lesdits composé(s) organique(s) liquide(s) est ou sont présent(s) dans des proportions valant de 1 à 40 % en poids par rapport au poids total de chaque composition le ou les contenant, et de préférence de 2 à 20 % en poids.

**10.** Procédé selon l'une quelconque des revendications précédentes, dans lequel au moins l'une des compositions mises en oeuvre contient également un ou plusieurs sel(s) de métal et/ou oxyde(s) de métal, choisi(s) de préférence parmi les sels et/ou oxydes de zinc, de manga-nèse, de fer, de cuivre ou d'aluminium, et mieux encore parmi le glyci-nate de zinc, le glycinate de manganèse, le chlorure de manganèse, le sulfate de fer et leurs mélanges.

**11.** Procédé selon la revendication précédente, dans lequel le ou les sel(s) de métal et/ou oxyde(s) de métal utilisé(s) représente(nt) de 0,001 % à 10 % en poids du poids total de la ou des composition(s) contenant ce ou ces sel(s) de métal et/ou oxyde(s) de métal, et de préférence de 0,05 % à 5 % en poids.

**12.** Procédé selon l'une quelconque des revendications précédentes, dans lequel aucune des compositions mises en oeuvre ne contient d'oxydant chimique.

**13.** Procédé selon l'une quelconque des revendications précédentes, comprenant une première étape consistant à appliquer sur les fibres kératiniques la composition tinctoriale (a), puis une deuxième étape consistant à appliquer sur les fibres kératiniques la composition (b).

**14.** Kit pour la teinture de fibres kératiniques, comportant au moins deux compartiments :

- un premier compartiment renfermant une composition tinctoriale (a) comprenant un ou plusieurs colorant(s) choisi(s) parmi les colorants chroméniques et les colorants chromaniques ;
- et un deuxième compartiment renfermant une composition (b) comprenant un ou plusieurs acide(s) polyphénolique(s) choisi(s) parmi l'acide tannique, l'acide gallique, l'acide ellagique et les sels de ces acides.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 102048670
- FR 2814943
- FR 2586913


### Non-patent literature cited in the description


- **CHARLES M. HANSEN.** Hansen solubility parameters A user's handbook. CRC Press, 2000, 167-185
- Handbook of Solubility Parameters and other cohesion parameters. CRC Press, 95-121
- Handbook of Solubility Parameters and other cohesion parameters. CRC Press, 95-121, 177-185
- **BAGDA, E ; FARBE LACK.** *The relation between surface tension and solubility parameter in liquids,* 1978, vol. 84, 212